# EUROPEAN PATENT APPLICATION

(11) **EP 1 704 842 A1**
(43) Date of publication of application: **27.09.2006**
(21) Application number: 04808161.6
(22) Date of filing: 27.12.2004
(51) Int. Cl.: A61F 13/514, A61F 13/49

(54) **PAPER DIAPER AND PAPER DIAPER PRODUCT PACKAGE**

(30) Priority: 15.01.2004 JP 2004007812
(71) Applicant: DAIO PAPER CORPORATION, Shikokuchuo-shi, Ehime 799-0402 (JP)
(72) Inventor: KANEDA, Masahiro, DAIO PAPER CONVERTING CO., LTD., Ehime 7990431 (JP)
(74) Representative: Hooiveld, Arjen Jan Winfried
(86) International application number: PCT/JP2004/019811
(87) International publication number: WO 2005/067853

(57) **Abstract**

In a disposable diaper in which a disposable diaper product main body is formed by at least a top sheet, a back sheet and an absorber interposed between the sheets, and a plurality of design prints are applied to a position which is visible from an outer portion of the disposable diaper product main body, in a width direction of the disposable diaper product main body from a waist opening end portion of a front body to a waist opening end portion of a back body, the disposable diaper is produced by applying the design prints so as to satisfy a relation "A > B ≥ 1/3A", in which a width of the disposable diaper product main body is set to A and a width of a surface of the design prints is set to B. A disposable diaper product package is produced by accommodating a plurality of the disposable diaper products having different designs within the same package.

## Description

### TECHNICAL FIELD

The present invention relates to a disposable diaper in which a disposable diaper product main body is formed by at least a tope sheet, a back sheet and an absorber interposed between both the sheets, and a design print is applied to a position which is visible from an outer portion of the disposable diaper main body, and a disposable diaper product package formed by accommodating a plurality of disposable diaper products within the same package.

### BACKGROUND ART

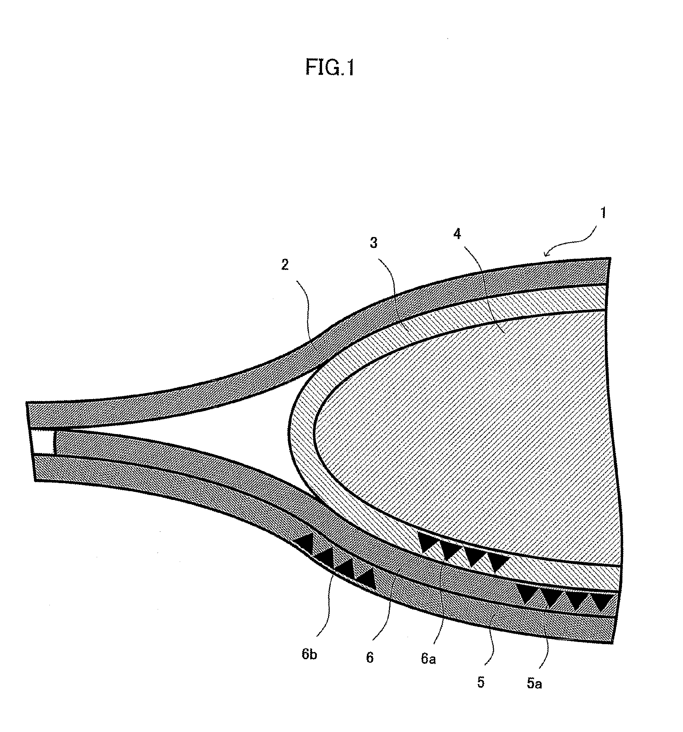

Various sizes and shapes of disposable diapers are produced in accordance with purposes, intended uses or the like, and are actually used for a lot of peoples from a nursling to an elderly person. However, basic structures thereof do not make much difference. For example, in a disposable diaper 1 shown by a cross sectional view of a main portion in FIG. 1, a disposable diaper product main body is constituted by a top sheet 2, an absorber 4 coated by a crepe paper 3 or the like and a back sheet 5 alphabetically from a side brought into contact with a body of a wearer (hereinafter, refer to "body side"). In this case, there have been known the disposable diapers in which a three-dimensional gather (not shown) is provided in the body side of the top sheet 2, a second sheet (not shown) is provided in an opposite body side of the top sheet 2, and a waterproof film 6 is provided in the body side of the back sheet 5, as occasion demands.

In the disposable diaper 1 mentioned above, for the purpose of applying a sense of beauty and an underwear feeling so as to intend to improve a quality, displaying a back and forth directions of the disposable diaper 1 so as to accommodate at a time of wearing, promoting an intellectual education and a potty-training of the nursling or the like, various design prints are generally applied to a position which is visible from an outer portion of the disposable diaper product main body, in a width direction of the disposable diaper product main body, from a waist opening end-portion of a front body to a waist opening end-portion of a back body. In this case, the design in the present invention means an image or a picture, a graphic, a character, a color itself and a combination thereof.

Conventionally, the design print is frequently applied to an outer surface of the back sheet 5 in view of easiness for the printing. However, since the design print surface applied in the manner mentioned above is brought into direct contact with the body or the cloth, there is a problem that unless the surface of the design print surface is protected by a coating or the like, the printed position is wet with sweat or grazed, whereby the ink is transcribed to the cloth or grazed by the cloth. Accordingly, the design print surface is recently set to a position which can not be directly touched from the external portion, specifically, a body side surface 5a of the back sheet 5, or a body side surface 6a or an opposite body side surface 6b of the waterproof film 6, as shown by a mark "▲" in FIG. 1 (refer to a reference, for example, Japanese Patent Application Laid-open No.2003-70838 A).

Further, in the disposable diaper, there has been known a disposable diaper provided with a design sheet (not shown) in which a desired design print is previously applied to a portion between the back sheet 5 and the absorber 4, or a portion between the back sheet 5 and the waterproof film 6, in addition to the disposable diaper in which the print is directly applied to the position of the disposable diaper product main body. However, in the case of the disposable diaper, a process for producing and installing the design sheet is independently necessary, whereby a production cost is widely increased due to an increase of a material cost and an increase of a number of the processes. Therefore, the former disposable diapers are more produced generally, and are provided to the market.

Conventionally, the design print mentioned above is printed to the predetermined position mentioned above of the disposable diaper product main body in accordance with various known printing methods such as a gravure printing, a flexographic printing and the like, in the production process of the disposable diaper 1.

However, since the design printed in the manner mentioned above is generally constituted by the same pattern having the same image, character or color in most cases, it is an actual condition that the disposable diaper or the disposable diaper product package is comprehended as an extremely simple and non-individual, in other words, non-distinctive commodity, in the case that a consumer views the disposable diapers having the design print mentioned above, particularly the disposable diaper product package formed by accommodating a plurality of disposable diaper products. An image of the consumer mentioned above is an important factor which reduces a commercial value of the disposable diaper, thereby affecting a sale promotion of the disposable diaper product.

The present invention is made by taking the actual condition mentioned above, and an obj ect of the present invention is to provided a disposable diaper and a disposable diaper product package, which can apply an image of an extremely varied commodity with personality, in other words, an attractive and distinctive commodity to the consumer, in the case that the consumer views the disposable diaper and the disposable diaper product package formed by accommodating the disposable diaper products in one package, and can be inexpensively produced.

### DISCLOSURE OF THE INVENTION

The obj ect of the present invention can be achieved by providing a disposable diaper in which a disposable diaper product main body is formed by at least a top sheet, a back sheet and an absorber interposed between the sheets, and a plurality of design prints are applied to a position which is visible from an outer portion of the disposable diaper product main body, in a width direction of the disposable diaper product main body from a waist opening end-portion of a front body to a waist opening end-portion of a back body, wherein the design prints are applied so as to satisfy a relation "A > B ≥ 1/3A", in which a width of the disposable diaper product main body is set to "A" and a width of a surface of the design prints is set to "B".

The obj ect of the present invention can be effectively achieved by providing a disposable diaper, wherein the plural patterns of design prints are structured such that the patterns of the adjacent print surfaces are printed by approximate colors close to related colors.

Further, the object of the present invention can be more effectively achieved by providing a disposable diaper, wherein the deign prints are applied to a position which does not directly touch with a body of a wearer of the disposable diaper in the disposable diaper product main body.

Further, the object of the present invention can be more effectively achieved by providing a disposable diaper, wherein the design prints are arranged in a curved manner.

Furthermore, the object of the present invention can be more effectively achieved by providing a disposable diaper product package, wherein a plurality of the disposable diapers recited in any one of the first to fourth aspects are accommodated within the same package.

Still further, the object of the present invention can be more effectively achieved by providing a disposable diaper product package, wherein the package is constituted by a transparent or semitransparent sheet material.

### EFFECT OF THE INVENTION

In the disposable diaper and the disposable diaper product package formed by accommodating a plurality of the disposable diapers within the same package, since the plural patterns of the design prints are applied to the position which is visible from the outer portion of the disposable diaper product main body, in the width direction of the disposable diaper product main body from the waist opening end-portion of the front body to the waist opening end-portion of the back body, and the different design prints are applied to the disposable diaper products from each other, it is possible to apply an image of an extremely varied commodity with personality, in other words, an attractive and distinctive commodity to the consumer viewing the disposable diaper product, whereby it is possible to promote a sale of the disposable diaper product.

Particularly, in the structure in which the patterns of the adjacent print surfaces in the plural patterns of the design prints are printed by the approximate colors close to the related colors, since the change of the design is made more definite, it is possible to make the effect more remarkable.

Since the deign prints are applied to the position which does not directly touch with the body of the wearer of the disposable diaper produce main body, there is no risk that the print portion is transcribed or grazed off.

Further, in the case that the design prints are arranged in the curvedmanner, it is possible to obtain a design with more variety, and it is possible to make the change of the design more definite. Accordingly, it is possible to make the effect mentioned above more remarkable.

Further, since the consumer can view the disposable diaper in accordance with the present invention having the design with more variety having the different patterns from the outer side of the disposable diaper product package, by constituting the disposable diaper product package by the transparent or semitransparent sheet material, it is possible to make the effect mentioned above more remarkable.

Furthermore, since the disposable diaper and the disposable diaper product package formed by accommodating a plurality of the disposable diaper products within the same package in accordance with the present invention, can be produced by adding some amount of equipment to the conventional production equipment, it is possible to widely reduce the production cost in comparison with the case of the conventional disposable diaper produced by using the design sheet.

### BRIEF DESCRIPTION OF THE DRAWINGS

FIG. 1 is a cross sectional view of a main portion of a general disposable diaper product main body;
FIG. 2 is a schematic side view of a printing process in accordance with an embodiment of the present invention;
FIG. 3 is a plan view showing an embodiment of a design printed by the process;
FIG. 4 is an exploded plan view of a disposable diaper product main body to which a design print is applied;
FIG. 5 is a perspective view of a pants type disposable diaper formed by the disposable diaper product main body;
FIG. 6 is a front view of a disposable diaper showing a first modified embodiment of the design print;
FIG. 7 is a front view of a disposable diaper showing a second modified embodiment of the design print;
FIG. 8 is a front view of a disposable diaper showing a third modified embodiment of the design print;
FIG. 9 is a front view of a disposable diaper in accordance with the other embodiment of the present invention;
FIG. 10 is a front view of a disposable diaper in accordance with further the other embodiment of the present invention; and
FIG. 11 is a perspective view of a disposable diaper product package in accordance with an embodiment of the present invention.

- 1, 11, 11a-11e, 11x, 11y, 11z: (pants type) disposable diaper
- 2: top sheet
- 3: crepe paper
- 4: absorber
- 5, 15: back sheet
- 6: waterproof film
- 20: printing process
- 21, 22, 23, 24, 25: printing apparatus
- 33: package
- 34: disposable diaper product package

### BEST MODE FOR CARRYING OUT THE INVENTION

A description will be in detail given below of contents of the present invention by exemplifying a pants type disposable diaper for a nursling (a baby) (hereinafter, refer simply to as "disposable diaper"). In this case, it goes without saying that the present invention is not necessarily limited to the following embodiment, but the structure of the present invention can be variously modified within the scope of the present invention.

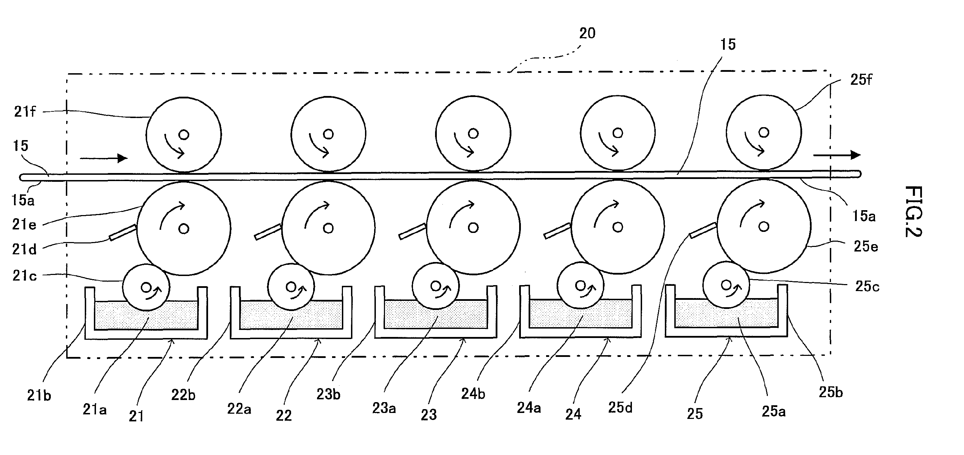

FIG. 2 is a schematic side view of a design printing process 20 in a producing process of a disposable diaper 11 in accordance with the present invention, and shows a state in which a back sheet 15 constituting an outermost surface of the disposable diaper 11 is printed by the printing process 20.

The back sheet 15 is combined with an independently fed top sheet after a design print is applied thereto, and is cut into a predetermined shape after an absorber is installed to an inner portion between both the sheets. However, since the producing process is well known in this field, a description thereof will be omitted.

The printing process 20 is constituted by five sets of gravure printing apparatuses 21, 22, 23, 24 and 25 having the same structure. The gravure printing apparatus 21 is constituted by an ink tank 21b accommodating a blue ink 21a, an ink roller 21c, a doctor blade 21d, a gravure cylinder 21e and an impression cylinder 21f. The blue ink 21a attached to the ink roller 21c is scrapedoff at a predetermined amount by the doctor blade 21d on the basis of a rotation of the respective rotating members 21c, 21e and 21f in directions of arrows, whereby a design having a desired pattern is printed on a body side surface 15a of the back sheet 15 fed between the gravure cylinder 21e and the impression cylinder 21f. In this case, since the gravure printing apparatuses 22, 23, 24 and 25 are structured in the same manner, the following description will be given by attaching the corresponding same reference numerals to the corresponding same constituting parts to the gravure printing apparatus 21.

In the present embodiment, there is employed a color design formed by using five kinds of colors and printing each of the colors in a rectangular shape. The five kinds of colors employ the blue ink 21a, a purple ink 22a, a green ink 23a, a red ink 24a and a navy blue ink 25a, and these inks are respectively accommodated in the ink tanks 21b, 22b, 23b, 24b and 25b so as to be set before the printing.

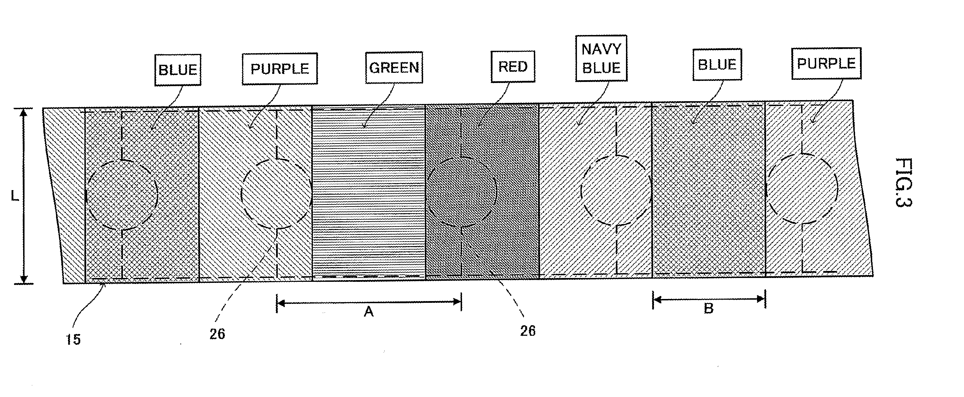

FIG. 3 is a plan view in which the back sheet 15 having the design printed by the printing process 20 is seen from the body side surface 15a. In FIG. 3, reference symbol "L" denotes a width of the back sheet 15, that is, a dimension in a longitudinal direction at a time of expanding the present disposable diaper 11 and viewing from a plane, reference symbol "A" denotes a product width of the present disposable diaper 11, and reference symbol "B" denotes a width of the design print, respectively. In this case, a dotted line 26 shows a cut position at a time when the back sheet 15 is cut into a predetermined product shape in the later process.

As illustrated, the color design prints of the rectangular pattern by the blue, purple, green, red and navy blue colors are continuously applied to the body side surface 15a of the back sheet 15 sequentially at the same cycle, by the gravure printing apparatuses 21 to 25.

Generally, the dimension L in the longitudinal direction is set to a range between 30-100 [cm], and the product width A is set to a range between 26 - 70 [cm]. In the present invention, the print width B is associated with the product width A, and is set in such a manner as to satisfy a relation "A > B ≥ 1/3A". This is because if the relation "B < 1/3A" is set, the design print surface becomes too fine and the same color design gets into the same disposable diaper product so as to be complicated, and if the relation "A < B" is conversely set, the disposable diaper forming the single color design print surface appears, and the color variation can not be obtained.

If the design print is applied as mentioned above, the adjacent disposable diapers 11 have the different designs from each other until the print finishes one cycle, so that it is possible to continuously produce the disposable diaper 11 having the design with more variety in the same printing process 20.

In the case that the design is applied only by the colors as in the present invention, it is desirable that the patterns of the adjacent print surfaces are printed by approximate colors close to related colors. In accordance with this structure, it is possible to output definite color variation and beauty, whereby it is possible to improve a commercial value of the disposable diaper.

Further, since the printing process 2 0 can be achieved by adding some amount of printing apparatus to the conventional printing process, it is unnecessary to arrange an independent process, whereby it is possible to inexpensively produce the disposable diaper 11.

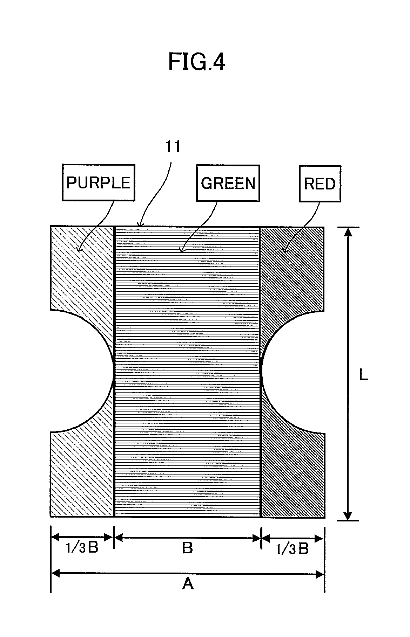

FIG. 4 is a plan view of a product main body of the disposable diaper 11 to which the design print is applied and which is finally cut into a predetermined shape as being expanded and seen from an outer side (an opposite body side), and corresponds to a cut portion of the back sheet 15 shown in an approximately center portion in FIG. 3. As illustrated, in the disposable diaper 11 in accordance with the present embodiment, there is applied such a design that the green print surface having the print width B is arranged in a vertically long shape in a center portion with respect to the product width A, and the purple print surface and the red print surface each having the print width 1/3B are symmetrically arranged in the respective side surfaces.

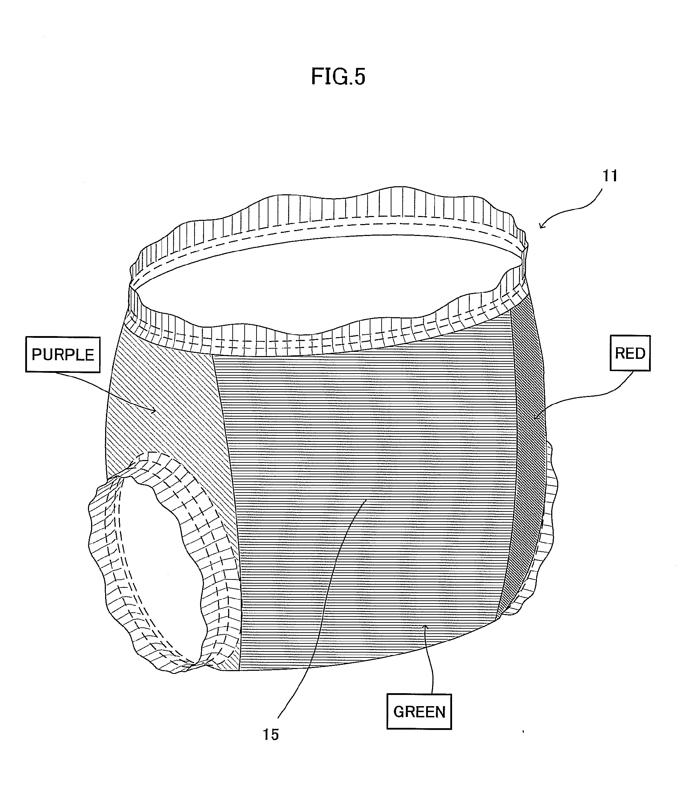

FIG. 5 is a perspective view at a time when a pants type disposable diaper 11 is finished by folding the disposable diaper product main body shown in FIG. 4 from the center portion in the longitudinal direction and welding the open side end portions.

As is well known, the back sheet 15 provided in the outermost layer of the disposable diaper 11 structures a body fluid impermeable back surface material, and the material generally employs a laminate unwoven fabric formed by laminating an unwoven fabric on a polyethylene sheet or the like, an unwoven fabric sheet substantially securing an impermeability by interposing a waterproofing film, or the like, in addition to a sheet material which does not transmit at least the body fluid, for example, an olefin resin sheet such as a polyethylene, a polypropylene or the like. However, since the back sheet is thin and has a transparency, the consumer can clearly view the design from the outer portion even if the design is applied to the body side surface 15a of the back sheet 15 as mentioned above.

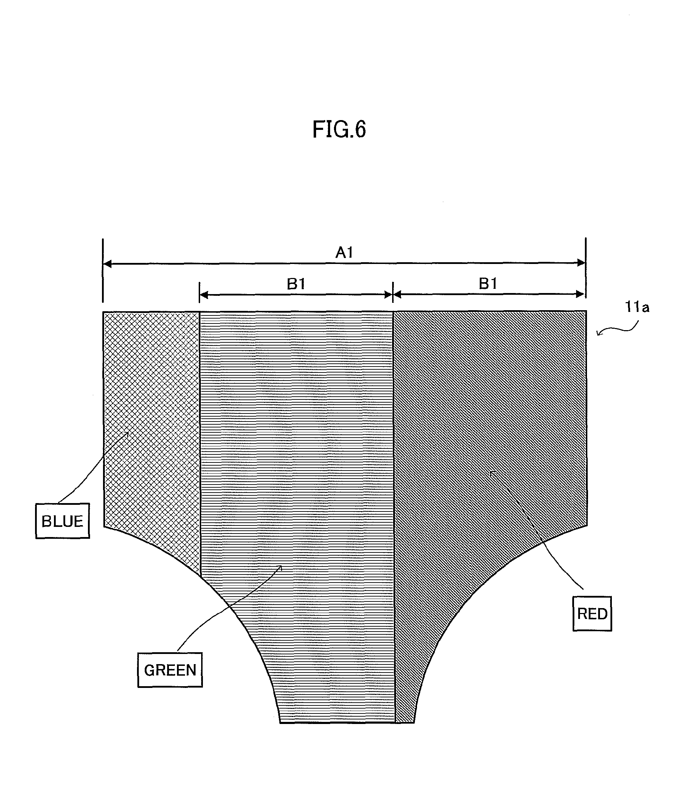

FIG. 6 shows a first modified embodiment of the disposable diaper in which the design mentioned above is constituted only by the colors, and shows a pants type disposable diaper 11a as seen from the front surface. As illustrated, in the present disposable diaper 11a, there is applied such a design that a green print surface having a print width B1 is arranged in a vertically long shape in an approximately center portion with respect to a product width A1, a red print surface having the print width B1 is arranged in a left side (a right side in the front view), and a blue print surface is arranged in a right side (a left side in the front view). In this case, in the present embodiment, the product width A1 is set to 374 [mm], and the print width B1 is set to 130 [mm]. Further, the product width and the print width can be optionally set in correspondence to a purpose and an intended use, for example, the product width A1 is set to 414 [mm] and the print width B1 is set to 150 [mm] in correspondence to a body length of the wearing nursling, or the like.

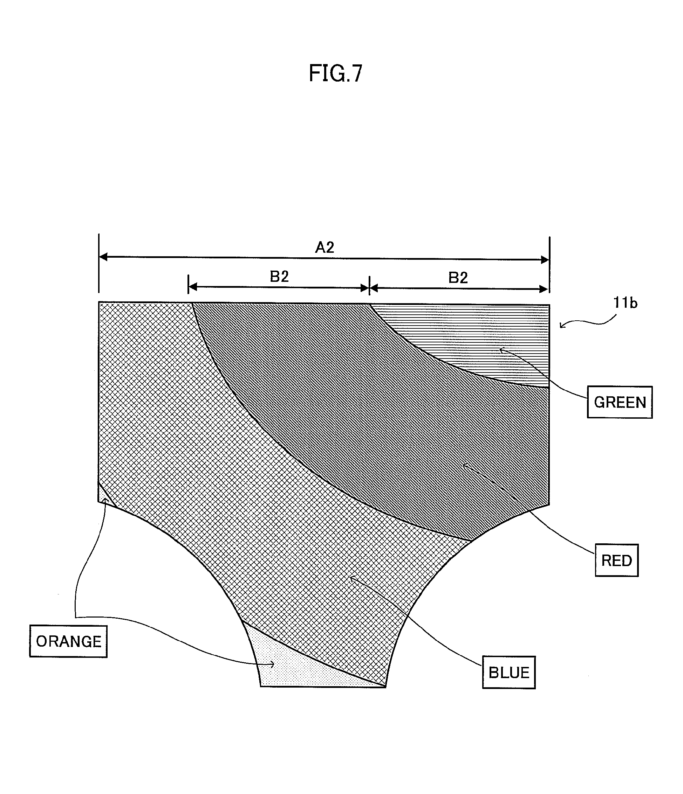

FIG. 7 shows a second modified embodiment of the disposable diaper in which the design is constituted only by the colors, and shows a pants type disposable diaper 11b as seen from the front surface. As illustrated, in the present disposable diaper 11b, there is applied such a design that a red print surface having a print width B2 is arranged in a curved shape in an approximately center portion with respect to a product width A2, a green print surface having the print width B2 is arranged in a left side (a right side in the front view), a blue print surface is arranged in a right side (a left side in the front view), and an orange print surface is arranged in adjacent to the blue print surface. In this case, in the present embodiment, the product width A2 is set to 374 [mm], and the print width B2 is set to 130 [mm].

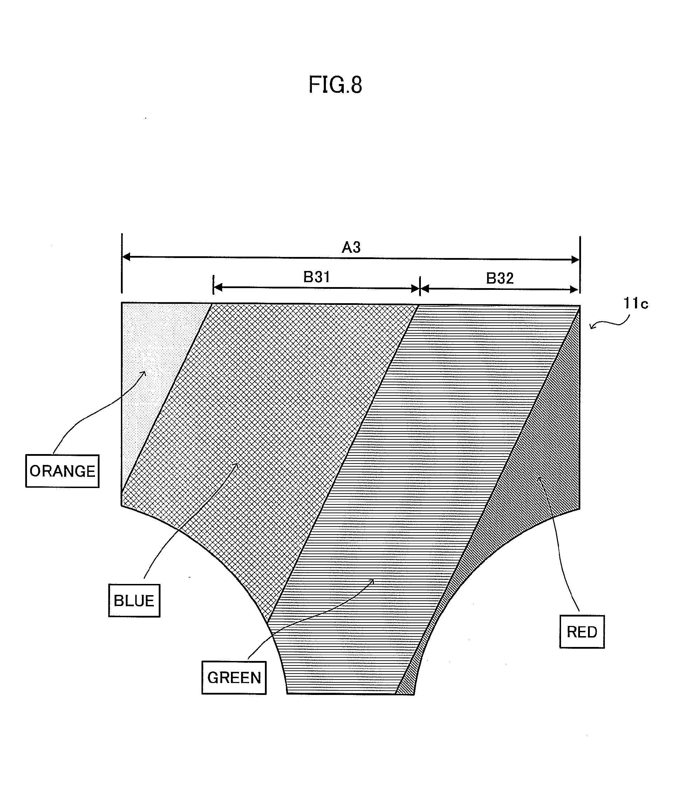

FIG. 8 shows a third modified embodiment of the disposable diaper in which the design is constituted only by the colors, and shows a pants type disposable diaper 11c as seen from the front surface. As illustrated, in the present disposable diaper 11c, there is applied such a design that a blue print surface having a print width B31 is arranged in a diagonal line shape in an approximately center portion with respect to a product width A3, a green print surface having a print width B32 is arranged in a left side (a right side in the front view), a red print surface is arranged in adjacent to the green print surface, and an orange print surface is arranged in a right side (a left side in the front view) of the blue print surface. In this case, in the present embodiment, the product width A3 is set to 414 [mm], the print width B31 is set to 180 [mm], and the print width B32 is set to 150 [mm].

In all of the modified embodiments, since the patterns of the adjacent print surfaces are printed by the approximate colors close to the related colors, and the design having more variety is formed, it is possible to obtain the same effect as that of the case of the disposable diaper 11 mentioned above.

The embodiments mentioned above correspond to the case in which the design is constituted only by the colors, however, the design can be replaced by various aspects.

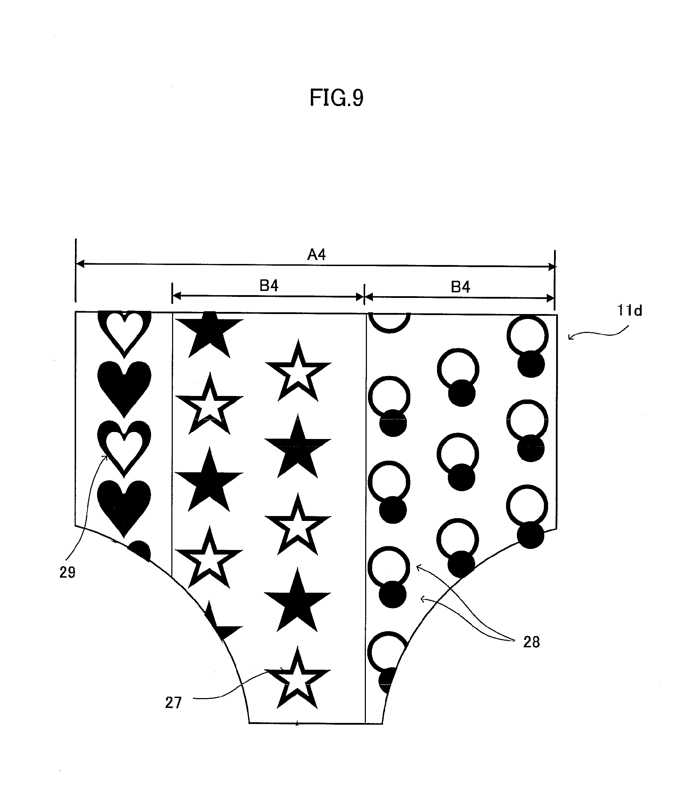

FIG. 9 shows the other embodiment in accordance with the present invention, and shows a disposable diaper 11d finished as a pants type in the same manner as seen from the front surface. As illustrated, in the present disposable diaper 11d, there is applied such a design that a print surface of a print width B4 having a star graphic 27 is arranged in a vertically long shape in an approximately center portion with respect to a product width A4, a print surface of the print width B4 having a circular graphic 28 is arranged in a left side (a right side in the front view), and a print surface having a heart graphic 29 is arranged in a right side (a left side in the front view) of the print surface having the star graphic 27. In this case, in the present embodiment, the product width A4 is set to 414 [mm], and the print width B4 is set to 150 [mm]. As mentioned above, the design of the present disposable diaper 11 can be constituted by a combination of the graphics.

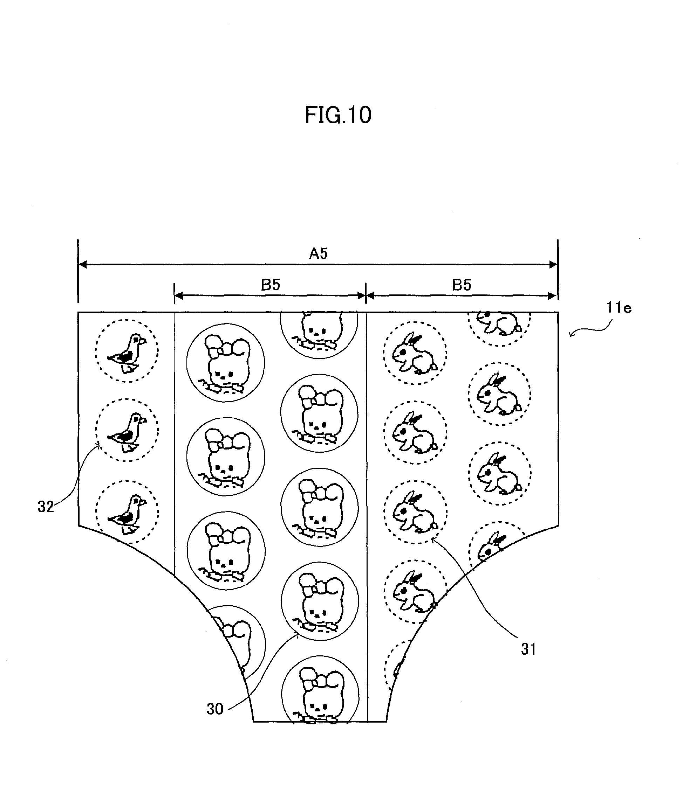

FIG. 10 shows further the other embodiment in accordance with the present invention, and shows a disposable diaper 11e finished as a pants type in the same manner as seen from the front surface. As illustrated, in the present disposable diaper 11e, there is applied such a design that a print surface of a print width B5 having a puppy image 30 is arranged in a vertically long shape in an approximately center portion with respect to a product width A5, a print surface of the print width B5 having a rabbit image 31 is arranged in a left side (a right side in the front view), and a print surface having a little bird image 32 is arranged in a right side (a left side in the front view) of the print surface having the puppy image 30. In this case, in the present embodiment, the product width A5 is set to 374 [mm], and the print width B5 is set to 150 [mm]. As mentioned above, the design can be constituted by a combination of the images.

As mentioned above, in the case that the design is constituted by the various graphs or the images such as animals or the like, it is possible to achieve an intellectual education purpose for the nursling wearing the disposable diaper.

In this case, all of the embodiments relate to the case that the design is applied to the body side surface of the back sheet, however, in the present invention, the design may be applied to the body side surface 6a of the waterproof film 6 or the opposite body side surface 6b, as shown by the mark "▲" in FIG. 1, or may be applied to a body surface side of a gather pressing sheet (not shown) provided in the lower side of the top sheet 2 as occasion demands.

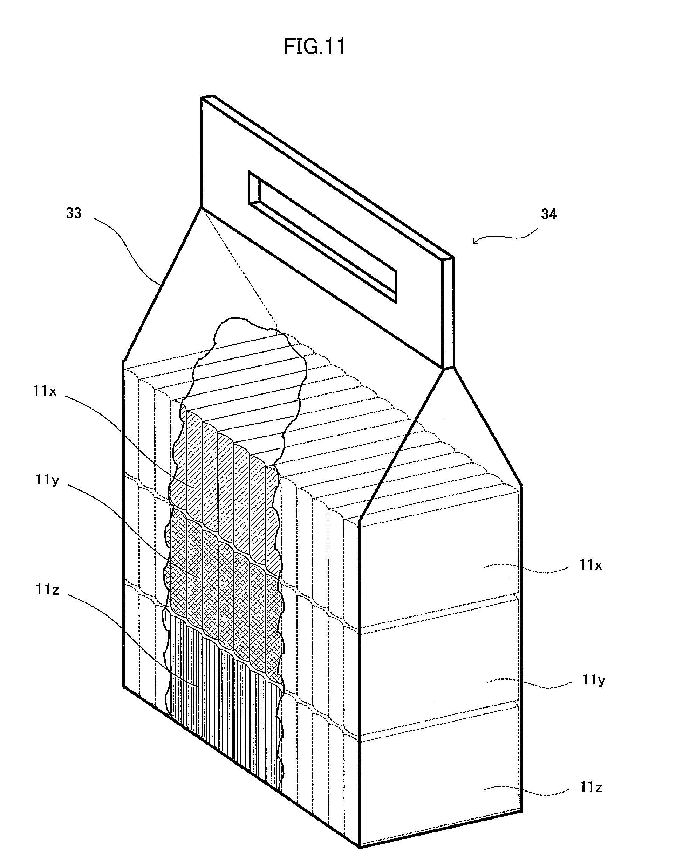

FIG. 11 is a perspective view of a disposable diaper product package 34 formed by aligning a plurality of three kinds of disposable diapers 11x, 11y and 11z produced via the printing process 20 and having different designs, and accommodating them in an inner portion of a package 33 while stacking them in three stages. Since the package 33 is formed by a transparent or semitransparent sheet material, the consumer can view the disposable diapers 11x, 11y and 11z accommodated in the inner portion from an outer side of the disposable diaper product package 34. As mentioned above, since the present disposable diaper product package 34 is constituted by accommodating the disposable diapers 11x, 11y and 11z respectively having the designs with the different patterns and with the more variety within one package 33, the present disposable diaper product package 34 tends to catch the eye of the consumer, and it is possible to apply the image of the commodity with a personality to the consumer. In this case, it goes without saying that it is possible to change the packaging aspect of the disposable diaper product package 34 to various aspects.

### INDUSTRIAL APPLICABILITY

The present invention is not limited to the pants type disposable diaper, but can be additionally applied to a tape type disposable diaper or a similar absorbable material. Further, it goes without saying that the printing method is not limited to the gravure printing, but can utilize various printing methods such as a flexographic printing or the like.

## Claims

1. A disposable diaper in which a disposable diaper product main body is formed by at least a top sheet, a back sheet and an absorber interposed between said top sheet and said back sheet, and a plurality of design prints are applied to a position which is visible from an outer portion of said disposable diaper product main body, in a width direction of said disposable diaper product main body from a waist opening end-portion of a front body to a waist opening end-portion of a back body, wherein said design prints are applied so as to satisfy a relation "A > B ≥ 1/3A", in which a width of said disposable diaper product main body is set to A and a width of a surface of said design prints is set to B.

2. A disposable diaper as claimed in claim 1, wherein said plural patterns of design prints are structured such that the patterns of adjacent print surfaces are printed by approximate colors close to related colors.

3. A disposable diaper as claimed in claim 1 or 2, wherein said deign prints are applied to a position which does not directly touch with a body of a wearer of said disposable diaper in said disposable diaper product main body.

4. A disposable diaper as claimed in any one of claims 1 to 3, wherein said design prints are arranged in a curved manner.

5. A disposable diaper product package, wherein a plurality of the disposable diapers as claimed in any one of claims 1 to 4 are accommodated within same package.

6. A disposable diaper product package as claimed in claim 5, wherein said package is constituted by a transparent or semitransparent sheet material.
